# EUROPEAN PATENT APPLICATION

(11) **EP 4 817 666 A1**
(43) Date of publication of application: **07.10.2026**
(21) Application number: 24896804.2
(22) Date of filing: 14.10.2024
(51) Int. Cl.: A61M 25/00, A61M 27/00

(54) **CATHETER FOR TRANSPORTING BIOLOGICAL FLUIDS AND SOLUTES THEREOF**

(30) Priority: 30.11.2023 ES 202330997
(71) Applicant: Neuroscience Innovative Technologies, S.L., 33428 Llanera Asturias (ES)
(72) Inventor: ÁLVAREZ MENÉNDEZ, Gabriel, 33428 Llanera Asturias (ES); MENÉNDEZ GONZÁLEZ, Manuel, 33428 Llanera Asturias (ES); PRADO CUETO, Catuxa, 43428 Llanera Asturias (ES); RODRÍGUEZ CAÑÓN, María, 33428 Llanera Asturias (ES)
(74) Representative: Polo Montañés, Carlos
(86) International application number: PCT/ES2024/070621
(87) International publication number: WO 2025/114618

(57) **Abstract**

The implantable catheter, with the ability to generate an autonomous flow of fluid therein, consists of a tubular body (1), in which there is defined an internal morphology (2) determining two independent conduits (3-3') defining two lumens which, in correspondence with one of the ends thereof, open at different points and therefore at different heights of the cavity in which it is located, with one conduit being longer than the other one, that is, the one that moves naturally in the cavity or system due to a pressure gradient existing along same. This pressure gradient is transmitted through the orifices of each of the conduits of the catheter. This means that, at a given time, the pressure may be different in each of the end orifices, generating an autonomous and non-actively forced flow through the catheter. This continuous flow of the biological fluid can be used to administer medicinal products in a controlled manner, filter the fluid, etc. The interposition of one or more valves in one or more lumens or the interposed devices can cause the flow to be predominantly unidirectional or change the speed of the fluid.

## Description

### FIELD OF THE ART

The present invention relates to a catheter particularly designed for implantation in the body.

### OBJECT OF THE INVENTION

The object of the invention is to provide a catheter that allows a completely autonomous flow of bodily fluid to be generated within a closed circuit, without the support of active flow systems, whether unidirectional or bidirectional, thereby also being able to facilitate the transport of diluted solutes from one point of the circuit to another.

### BACKGROUND OF THE INVENTION

Catheters are used in clinical or preclinical setting as platforms for drawing or introducing physiological or pathological fluids, or drugs. They act as lines for communication between the body and the outside.

The fluids circulating within them are actively forced artificially, by aspiration or infusion, whether by a syringe or a small motor, as in peristaltic pumps (e.g., infusion pumps, implantable pumps, haemodialysis pumps, etc.), or naturally due to the difference in pressure between the fluid inside the body and the external pressure, as in cerebrospinal fluid drainage or blood draw.

Diluted molecules in a fluid within a tubular structure can be transported from one point to another either because there is a forced directional flow of the fluid or, in the case of a static or oscillating fluid, because the displacement of these molecules is caused solely or predominantly in one direction due to differences in molecular concentration, with this process being much slower.

Unlike this purely communicative function of the catheter, achieving a non-actively forced flow of bodily fluids within a closed system such as a catheter could be of interest for applications such as filtration, clearance, or the administration of drugs as the fluid passes through. It is desirable to avoid the use of electromechanical equipment or to change the pressures and volumes of bodily fluids.

The applicant is unaware of the existence of catheters that allow autonomous flow to be generated within them to carry out applications of this type for CSF or any other type of bodily fluid. Therefore, the closest known prior art for CSF is presented below, but this issue extends to other bodily fluids.

Simple or single-lumen intrathecal catheters:
Access to the subarachnoid space, bypassing the blood-brain barrier, can be achieved in the cranial region by reaching the ventricles or in the spinal region, usually the lumbar region. The latter is known as the intrathecal access route and is commonly used for the administration of certain analgesic drugs or for the drainage of cerebrospinal fluid (CSF). Both accesses can be used for drug administration, CSF drainage, and pressure monitoring using different types of catheters.

At the ventricular level, a common access is the so-called Ommaya reservoir. This is a long-term implant in which a subcutaneous reservoir is placed in the scalp and a catheter connects this reservoir to one of the cerebral ventricles, providing direct access to the CSF.

Ventricular catheters are also marketed for the treatment of hydrocephalus; these connect the inside of the ventricle to the outside, the peritoneum, or the atrium of the heart to control excess CSF.

Enclear Therapies is developing an extracorporeal CSF filtration and monitoring system, featuring two subcutaneous access ports that reach the subarachnoid space by means of catheters in the ventricular and lumbar regions. When both are connected to an active extracorporeal pumping system, they close the circuit through which the CSF circulates.

Alcyone Therapeutics is developing a new drug release system for the central nervous system called The ThecaFlex DRx^{™}, by means of a catheter inserted intrathecally and a subcutaneous reservoir. It is solely a bolus drug release system. Details of the catheter are not known.

There are several simple catheters used for intrathecal (lumbar) drug administration by means of implantable infusion pumps, such as the Ascenda catheter, for example, which is coupled to the Synchromed pump, with both products being from Medtronic.

### Double- or dual-lumen intrathecal catheter:

Experimental double-lumen catheters used in neurology are known to exist, such as the Neurapheresis^{™} system from Minnetronix Neuro to treat acute subarachnoid haemorrhages by means of extracorporeal filtration of the blood present in the cerebrospinal fluid.

This is a coaxial-design catheter in which a narrower, longer lumen is located inside a second, wider and shorter lumen. In this way, the CSF aspiration and return ports are distinguished by height within the spinal canal once the CSF has passed through an active extracorporeal peristaltic pumping and filtration system to remove blood from it. The catheter communicates the intrathecal space (at two different heights) to the external filtration platform. The different heights of the ports aim to prevent the aspiration of freshly returned CSF, thereby avoiding the filtration of CSF that has already been filtered. It is a short-term implant, with Neurapheresis sessions lasting up to 72 hours in a hospital setting, according to the PILLAR-XT clinical trial.

There is another double-lumen catheter, as part of the active fluid exchange system called IRRAflow, which is a catheter implanted at the intraventricular level to treat acute critical neurological conditions in a hospital setting. It was designed to allow active intraventricular fluid exchange, allowing the irrigation of physiological fluids and the drainage of pathological material (e.g., blood). It is a double-lumen catheter which, as a result of its connection to an external pump, allows CSF drainage through one lumen and fluid irrigation through the other. Both lumens end at the same height at a distal level. The fluid flow is active as a result of the external pump, not the catheter itself.

As for intellectual property registrations relating to devices of this type, the following are worth noting:
Patent ES2694698A1 relates to an implantable device for the removal of toxic molecules from cerebrospinal fluid. It consists of a double-lumen catheter in which the lumens are of equal length, which catheter has one free end in contact with the cerebrospinal fluid (CSF), while the other end has a capsule with two chambers separated by an intermediate partition.

The reality is that contrary to what is described in said patent, the applicant has been able to verify experimentally that the fact that the lumens are of equal length at the ends in contact with the CSF prevents any autonomous movement of the CSF from occurring, as the CSF pressure would be equal at both ends and CSF would attempt to enter both orifices equally, thereby eliminating the possibility of generating any movement within the device.

To achieve this, it is necessary to generate a pressure difference between the tips of the tubes that passively allows autonomous movement of the CSF within the tubes.

Although US Patent US2021386981A1, by Minnetronix Neuro, discloses a series of catheter designs the tubes of which have different lengths, these are assisted by a pump connectable between the two lumens to induce CSF flow, aspirating endogenous CSF through one of the lumens, conditioning it externally, and returning the treated CSF through the second lumen at a different height from the first. The CSF flow is actively forced by a peristaltic pump, which is essential to achieve the objective of the described patent. Therefore, the different heights of the lumens do not aim to generate passive CSF flow, but rather to prevent the CSF that has been freshly returned from being re-aspirated and thereby maximising the endogenous volume that is ultimately conditioned.

This same document US2021386981A1 discusses the turbulence generated at the outlet of the end of the return catheter, which aims to improve the mixing of conditioned and unconditioned (or endogenous) CSF, helping to reduce and homogenise the concentration of the target molecule in the total volume. It is described that this turbulence can be enhanced to a greater or lesser extent depending on different pump configurations (e.g., high-pressure injection) and catheter configurations (e.g.. multiple ports, balloons, different textures, etc.).

In any event, and as previously stated, this is not a passive device, as it uses a peristaltic pump to create CSF flow through the catheter; therefore, the different lengths of the lumens do not aim to generate passive CSF flow within the catheter.

In the case of US Patent US11123483B2, the catheter of which is that of the IRRAflow system, the aim is also not to produce an autonomous CSF or fluid flow, as it needs to be connected to a peristaltic pump for fluid exchange.

Accordingly, there is no known catheter in the field of CSF or other bodily fluid interventions that allows an autonomous flow to be generated within a closed system without the need for assistance from other components such as electromechanical pumps.

### DESCRIPTION OF THE INVENTION

The intrathecal or intraventricular catheter proposed by the invention fills in the technical void described above based on a simple yet highly effective solution.

To that end, the catheter of the invention consists of a doble-lumen catheter, conceived to generate a closed circuit, which allows a flow of CSF or other bodily fluids to be generated and the effective transport of molecules without the need for assistance from other active components such as electromechanical pumps.

Its structuring likewise allows other components, such as reservoirs and/or ports, to be placed in the closed circuit, and when such components are located subcutaneously or outside the body, they allow interaction with the CSF or other moving bodily fluids in different ways.

More specifically, the catheter consists of a tubular body, in which there is defined an internal morphology determining two independent conduits, defining two lumens. To generate movement, the fluid must have a different instantaneous pressure at each of the orifices of the lumens. This is achieved by positioning the terminal ends of the lumens at different heights within the same cavity or system of bodily fluids that exhibit a spatio-temporal variation in internal pressures.

In the case of the CSF, this allows said CSF to move naturally within the spinal canal due to a pressure gradient existing along same. Said pressures are transmitted through the orifices of each of the conduits of the catheter. This instantaneous pressure difference at their end orifices is what would allow a net flow other than zero within the catheter.

Based on this structuring, the catheter of the invention can be specifically configured as a closed circuit for different applications, in which CSF or other bodily fluids are displaced through the conduits by flowing naturally and not being actively forced. These fluids can be treated within the closed circuit itself, either by administering substances injected into reservoirs or ports in series and/or by modifying the composition of the fluid by means of using filters or membranes.

According to another feature of the invention, non-return systems may optionally be installed in the two conduits defined within the catheter to promote flow in one direction, or bidirectional movement (wave motion) may be established due to possible pulsatility when said systems are not present. In this regard, provision has been made for the inclusion of a valve system consisting of one or more valves arranged in one or both lumens of the catheter. In the latter case, they would be placed in opposite directions in each of the lumens, such that they can act synergistically to enhance fluid flow while at the same time favouring the inflow and outflow to a device connected in series predominantly in one of the directions.

Similarly, the physicochemical characteristics of the catheter may also be modified in terms of its composition, dimensions, or geometry, so as to enhance the flow of CSF or the effective transport of solutes diluted therein.

### DESCRIPTION OF THE DRAWINGS

To complement the description made below and for the purpose of helping to better understand the features of the invention according to a preferred practical embodiment thereof, a set of drawings is attached as an integral part of said description in which the following is depicted in an illustrative and non-limiting manner:
Figure 1 shows a perspective view of an intrathecal catheter carried out according to the object of the present invention.
Figure 2 shows a section view of the catheter of the previous figure, with a possible geometry for same.
Figure 3 shows a schematic drawing of the implantation mode of the catheter of the invention.

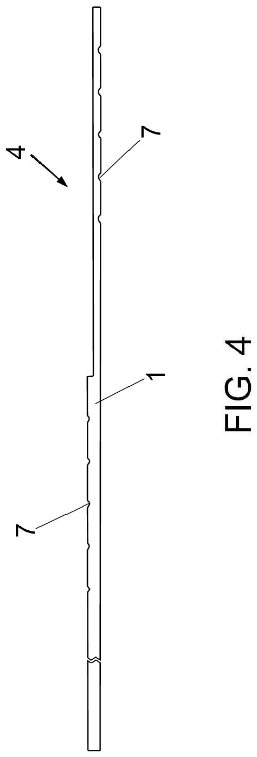

Figure 4 lastly shows a side elevational view of an embodiment variant of the catheter, which includes side orifices on both independent conduits.

### PREFERRED EMBODIMENT OF THE INVENTION

In view of the described figures, it can be seen how the device of the invention consists of a tubular body (1), having a length adapted to its final application, with a uniform or variable diameter, and in which there is established an internal morphology (2) determining two independent conduits (3-3') inside said tubular body, defining a doble-lumen catheter.

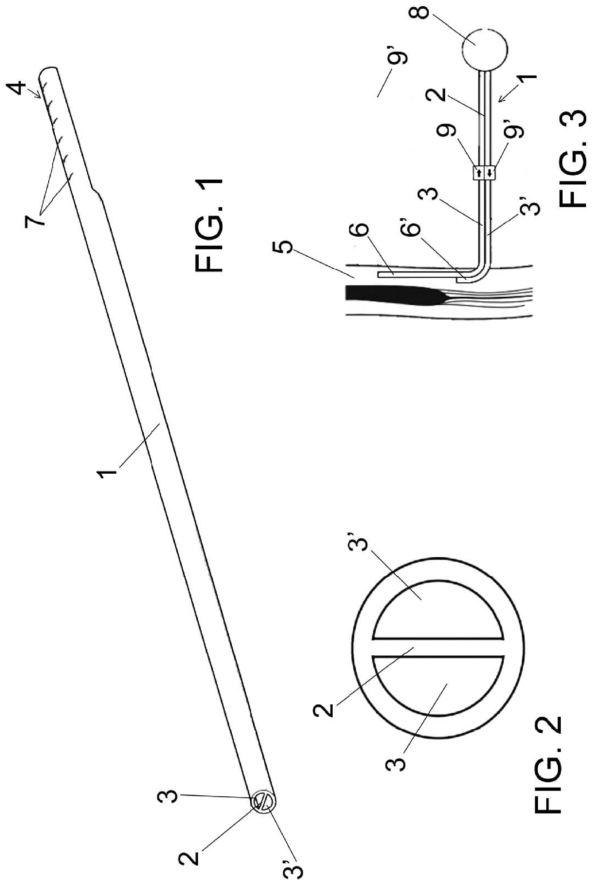

Therefore, according to the essence of the invention, and as can be seen in Figures 1 and 3, the independent conduits (3-3') of the end (4) of the catheter to be implanted, for example in the intrathecal region (5), open at different points (6-6') suitably spaced from one another. The outlet/inlet (6-6') of the conduits can be made by means of a single orifice at the end (4) and/or by means of additional side orifices (7) according to a suitable distribution.

Going back to Figure 3, in the case of the CSF, this allows said CSF to move naturally within the spinal canal or intrathecal region (5) due to a pressure gradient existing along same, where these pressures are transmitted through the orifices (6-6') of each of the conduits (3-3') of the catheter, the difference of which generates a natural flow of said CSF through the conduits. Said conduits can be connected in series at their opposite end to one or more elements (8), which may be a reservoir or port, to administer substances, and/or filters or membranes.

As for the internal morphology (2) determining the two conduits (3-3'), it can be indistinctly symmetrical, as is apparent in the figures, or asymmetrical, without this affecting the essence of the invention.

Lastly, it should be noted that, given that the movement of the CSF or other bodily fluids can naturally be bidirectional (wave-like), either the conduits (3-3') may be equipped with internal or external non-return systems (9-9') based on one or more valves arranged at any height in one or both lumens of the catheter to force the flow in one direction, or they may act synergistically to enhance fluid flow while at the same time favouring flow predominantly in one direction. The position and frequency of these non-return systems may vary in order to adjust the speed of the flow.

Therefore, this results in a catheter which, without the support of active flow systems, allows a fully autonomous movement of cerebrospinal fluid (CSF), whether unidirectional or bidirectional, to be generated within a closed circuit. This can be extended to other bodily fluids that exhibit pulsatility and may have different pressures at different heights of the cavity or system in which they are located.

## Claims

1. A catheter for the transport of biological fluids and solutes thereof, intended to generate an autonomous and non-actively forced movement of the bodily fluid within a closed circuit through said catheter, being **characterised in that** it consists of a tubular body (1), in which there is established an internal morphology (2) determining two independent conduits (3-3') inside said tubular body, wherein said conduits open at points (6-6') longitudinally spaced from one another in correspondence with the end (4) of the catheter intended to be implanted in the intrathecal region (5) or in another body cavity, the opposite end of said catheter having been provided to be connected in series through the independent conduits (3-3') to one or more elements (8), such as reservoirs or ports, flow enhancement systems, filters, or membranes (8), defining a passive bodily fluid flow system for drug administration or fluid filtration.

2. The catheter for the transport of biological fluids and solutes thereof according to claim 1, **characterised in that** the conduits (3-3') are equipped with an internal or external non-return system (9-9') determining the direction of flow and an effective transport of solutes predominantly in one direction in one or both conduits, wherein there can be one or more non-return systems or valves duly spaced from one another.

3. The catheter for the transport of biological fluids and solutes thereof according to claim 1, **characterised in that** the tubular body (1) has a uniform diameter.

4. The catheter for the transport of biological fluids and solutes thereof according to claim 1, **characterised in that** the tubular body (1) has a variable diameter.

5. The catheter for the transport of biological fluids and solutes thereof according to claim 1, **characterised in that** the end (4) of the catheter intended to be implanted in body cavities filled with biological fluids from any system in the body, such as the intrathecal space (5), for example, or others such as arteries or veins, has side inlets (7) suitably distributed around the opening (6-6') of the conduits (3-3').

6. The catheter for the transport of biological fluids and solutes thereof according to claim 1, **characterised in that** the internal morphology (2) determining the two conduits (3-3') exhibits symmetry.

7. The catheter for the transport of biological fluids and solutes thereof according to claim 1, **characterised in that** the internal morphology (2) determining the two conduits (3-3') has an asymmetrical distribution.
